# EUROPEAN PATENT SPECIFICATION

(11) **EP 0 638 302 B1**
(45) Date of publication and mention of the grant of the patent: **10.11.1999**
(21) Application number: 94305173.0
(22) Date of filing: 14.07.1994
(51) Int. Cl.: A61F 13/15

(54) **Sanitary napkin**
Monatsbinde
Serviette hygiénique

(30) Priority: 20.07.1993 JP 3951293
(43) Date of publication of application: 15.02.1995
(73) Proprietor: UNI-CHARM CORPORATION, Kawanoe-shi Ehime-ken (JP)
(72) Inventor: Wada, Mitsuhiro, Kawanoe-shi, Ehime-ken (JP); Kondo, Hideki, Kawanoe-shi, Ehime-ken (JP); Shimizu, Shingo, Mitoyo-gun, Kagawa-ken (JP)
(74) Representative: Murgatroyd, Susan Elizabeth

(56) References cited:
- EP-A- 0 581 258
- WO-A-92/07536
- WO-A-93/10733
- WO-A-93/12745
- WO-A-93/12747
- WO-A-93/19711
- WO-A-94/02095

## Description

The present invention relates to a sanitary napkin for treatment of menstruation.

Conventionally known sanitary napkins are provided with pockets or side flaps in laterally opposite side portions thereof, and further with elastic members disposed on the side flaps in an effort to prevent side leakage of body exudates. In Japanese Utility Model Laid-open No. Hei 2-26937 which is one known example, elastic strands are fixed longitudinally of side flaps to position in opposite sides of a core so that a bank function is provided to prevent leakage to outside.

A sanitary napkin is pressed against a local portion of a user in its use, generally assisted by tight shorts. This creates a problem in conventional napkins having elastic members on side flaps thereof in that those members are pushed deeply into a user's skin which can provide unexpected pain or discomfort to the user. The above known technique attempts to solve this problem by providing elasticity in a thickness direction of the strands. However, using this technique it is difficult to provide a complete solution because those strands project from a topsheet. In another known construction having side flaps which cover portions of a core, there is a problem that the mense absorbing rate is reduced in the covered portions.

WO-A-93 12747 discloses a sanitary napkin having a liquid-permeable topsheet, a liquid-impermeable backsheet and an absorbent core disposed between the topsheet and backsheet, with the sheets extending outwardly from the core to form side flaps. Each side flap has an elastomeric laminated member folded so as to be secured to the inner and outer surfaces of the side flap and has an unsecured portion or barrier cuff which is allowed to stand-up away from the side flap. A spacing member is provided to ensure that the barrier cuff does not collapse. In an alternative embodiment, the flap may be folded back upon itself at each end of the sanitary napkin and secured with an end seal.

The present invention is directed toward solving the above problems by providing a supplementary flap comprising an elastic band-like sheet on a top surface of each side flap which extends from a side edge of a core for contacting a user's skin.

Accordingly, the invention consists in a sanitary napkin comprising:
a liquid-permeable topsheet;
a liquid-impermeable backsheet;
an absorbent core having a pair of side edges, said core being interposed between said topsheet and said backsheet so that at least said backsheet extends outwardly from each side edge of said absorbent core to form a side flap;
an elastic supplementary flap disposed on a top surface of each said side flap, said supplementary flap extending longitudinally along said top surface and being stretched longitudinally of said napkin, said supplementary flap being secured to said top surface such that a side edge thereof forms a free edge, characterised in that said supplementary flap consists of a monolithic elastic sheet outwardly bi-folded to form a top portion and a bottom portion, said bottom portion having a top surface and a bottom surface being secured to said top surface of the side flap at said bottom surface thereof, said top portion being secured to said top surface of the bottom portion at opposite end portions of said top surface thereof.

When the sanitary napkin as thus constructed is pressed against a local portion of a user, the supplementary flaps secured to the side flaps in their stretched state tightly fit against the user's skin so that side leakage of menses are prevented. The supplementary flaps are not likely to be pushed deeply into the user's skin. Because the supplementary flaps do not cover the core, it does not affect the absorbing rate of the core.

The invention will be described in more detail with reference to the accompanying drawings, in which:-
Fig. 1 is a partly cut-away perspective view of a sanitary napkin.
Fig. 2 is a sectional view taken along a line II-II of Fig. 1.
Fig. 3 is a sectional view taken along a line III-III of Fig. 1.

A sanitary napkin in accordance with the present invention will be explained below in detail with reference to the accompanying drawings.

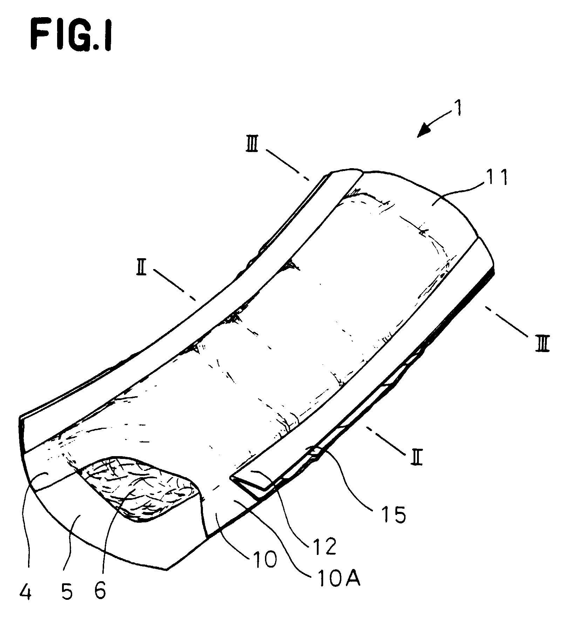

Figs. 1, 2, and 3 are a partly cut-away perspective view, a sectional view taken along a line II-II of Fig. 1, and a sectional view taken along a line III-III of Fig. 1, respectively. A napkin 1 comprises a liquid-permeable topsheet 4, a liquid-impermeable backsheet 5 and an absorbent core 6 interposed between the topsheet 4 and the backsheet 5. The topsheet 4 and the backsheet 5 respectively extend outwardly from opposite side edges and opposite end edges of the core 6 and respective extensions thereof are joined to each other by securement lines 18 of hotmelt type of adhesives or by heat-bonding to form side flaps 10 and end flaps 11.

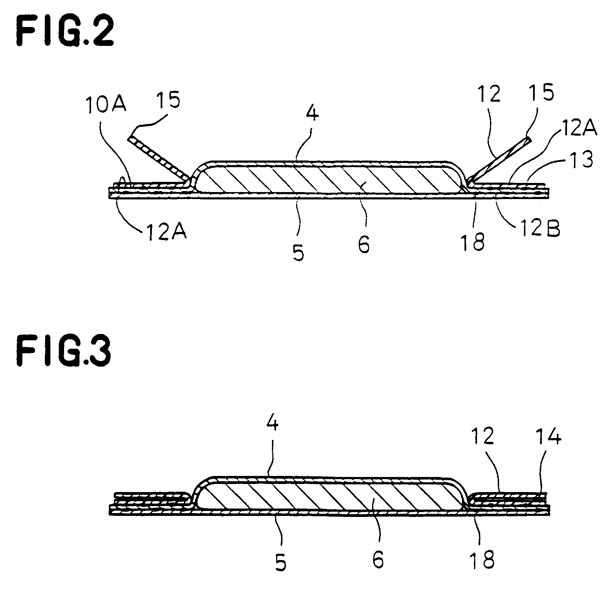

Each of the side flaps 10 has a supplementary flap 12 on its top surface 10A. The supplementary flap 12 comprises a band-like elastic sheet which is outwardly bi-folded to direct its top surface 12A inwardly so as to present a V-shape in cross section thereof which directs outwardly. The elastic sheet is stretched longitudinally of the napkin 1 and a bottom (proximal) surface 12B of a lower sheet portion as defined by the bi-folding of the elastic sheet is in its stretched state adhered to the side flap 10 by securement lines 13 of hotmelt type of adhesives (See Fig. 3). A top surface 12A of the sheet is secured onto itself by securement lines 14 in opposite end portions of the sheet. Accordingly, an outer side (distal) edge 15 of a longitudinal central portion of the sheet is rendered free to be elastically stretchable and contractable. When the napkin 1 is inwardly bent, e.g. when it is pressed against a local portion of a user, the outer side edge 15 of the supplementary flap 12 elastically contracts and is spaced away from the top surface 10A of the flap 10 to readily come into contact with the user's skin. The configurations of the outer edge as spaced away from the top surface are illustrated in Figs. 1 and 2.

The supplementary flap 12 may comprise an elastic plastic sheet, a non-woven fabric, or a rubber sheet. Those sheet materials are liquid-resistant, preferably liquid-impermeable, more preferably air-permeable and liquid-impermeable. Those sheet materials preferably have friction coefficients higher than that of the topsheet 4 to help prevent a top surface of the napkin 1 from slipping on the user's skin. The topsheet 4, the backsheet 5 and the absorbent core 6 may comprise conventionally used materials for sanitary napkins, respectively. Heat-bonding technologies as well as glues and adhesives such as hotmelt type of adhesives can be employed in securing any of those sheets.

The sanitary napkin in accordance with this invention has an elastic supplementary flap comprising a band-like sheet and disposed on a side flap so that when worn by a user it provides a comfortable feeling to the user during use and is not likely to be pressed deeply into the user's skin. Since the supplementary flap does not cover the absorbent core, it does not affect the absorbing rate of the absorbent core.

## Claims

1. A sanitary napkin comprising:
a liquid-permeable topsheet (4);
a liquid-impermeable backsheet (5);
an absorbent core (6) having a pair of side edges, said core (6) being interposed between said topsheet (4) and said backsheet (5) so that at least said backsheet (5) extends outwardly from each side edge of said absorbent core (6) to form a side flap (10);
an elastic supplementary flap (12) disposed on a top surface (10A) of each said side flap (10), said supplementary flap (12) extending longitudinally along said top surface (10A) and being stretched longitudinally of said napkin, said supplementary flap (12) being secured to said top surface (10A) such that a side edge thereof forms a free edge, characterised in that said supplementary flap (12) consists of a monolithic elastic sheet (12) outwardly bi-folded to form a top portion and a bottom portion, said bottom portion having a top surface and a bottom surface being secured to said top surface (10A) of the side flap (10) at said bottom surface thereof, said top portion being secured to said top surface of the bottom portion at opposite end portions of said top surface thereof.

2. A sanitary napkin according to Claim 1, wherein the bi-folding of said elastic sheet (12) defines a V-shape cross section including an apex, the apex being disposed inwardly.

3. A sanitary napkin according to Claim 2, wherein the apex is located adjacent the side edge of said absorbent core (6).

4. A sanitary napkin according to claim 1, 2 or 3, wherein the width of said top portion is substantially the same as that of said bottom portion.

## Patentansprüche

1. Monatsbinde, die folgendes umfasst:
eine flüssigkeitsdurchlässige Oberschicht (4);
eine flüssigkeitsundurchlässige Unterschicht (5);
einen absorbierenden Kern (6) mit einem Paar von Seitenkanten, wobei der Kern (6) zwischen der Oberschicht (4) und der Unterschicht (5) gelagert ist, so dass wenigstens die Unterschicht (5) sich von jeder Seitenkante des absorbierenden Kerns nach außen erstreckt, um einen Seitenflügel (10) zu bilden;
einen elastischen Hilfsflügel (12), der auf einer oberen Oberfläche (10A) von jedem Seitenflügel (10) angeordnet ist, wobei der Hilfsflügel (12) sich längs entlang der oberen Oberfläche (10A) erstreckt und längs von der Binde gestreckt ist, wobei der Hilfsflügel (12) an der oberen Oberfläche (10A) so befestigt ist, dass eine Seitenkante davon eine freie Kante bildet, dadurch gekennzeichnet, dass der Hilfsflügel (12) aus einer monolithischen elastischen Schicht (12) besteht, die auswärts doppelt gefaltet ist, um ein Oberteil und ein Unterteil zu bilden, wobei das Unterteil eine obere Oberfläche und eine untere Oberfläche hat, die an der oberen Oberfläche (10A) des Seitenflügels (10) an der unteren Oberfläche davon befestigt ist, wobei das Oberteil an der oberen Oberfläche des Unterteils an entgegengesetzten Endteilen der oberen Oberfläche davon befestigt ist.

2. Monatsbinde nach Anspruch 1, in der das doppelte Falten der elastischen Schicht (12) einen Ouerschnitt mit V-Gestalt definiert, der eine Spitze einschließt, wobei die Spitze nach innen angeordnet ist.

3. Monatsbinde nach Anspruch 2, in der die Spitze neben der Seitenkante des absorbierenden Kerns (6) angeordnet ist.

4. Monatsbinde nach Anspruch 1, 2 oder 3, in der die Breite des Oberteils im wesentlichen dieselbe wie die des Unterteils ist.

## Revendications

1. Serviette sanitaire comportant:
une nappe supérieure perméable au liquide (4);
une nappe de fond imperméable au liquide (5);
un noyau absorbant (6) comportant une paire de bords latéraux, ledit noyau (6) étant interposé entre ladite nappe supérieure (4) et ladite nappe de fond (5) de telle façon que la nappe de fond (5) au minimum s'étend extérieurement depuis chaque côté latéral dudit noyau absorbant (6) pour former un volet latéral (10);
un volet élastique supplémentaire (12) disposé en une surface supérieure (10A) de chaque dit volet latéral (10), ledit volet supplémentaire (12) étant en extension longitudinale au long de ladite surface supérieure (10A)et allongé en sens longitudinal de ladite serviette, ledit volet supplémentaire (12) étant fixé à ladite surface supérieure (10A) de façon telle qu'un bord latéral forme un bord libre, caractérisé en ce que ledit volet supplémentaire (12) consiste d'une nappe élastique monolithique (12) pliée double extérieurement pour former une portion supérieure et une portion inférieure, ladite portion inférieure ayant une surface supérieure et une surface inférieure fixées à ladite surface supérieure (10A) du volet latéral (10) en sa surface inférieure, ladite portion supérieure étant fixée à ladite surface supérieure de la portion inférieure aux portions d'extrémités opposées de ladite surface supérieure.

2. Serviette sanitaire selon la revendication 1 , suivant laquelle le pliage exécuté double de ladite nappe élastique (12) définit un élément comportant une coupe en Vé y compris un sommet, le sommet étant situé intérieurement.

3. Serviette sanitaire selon la revendication 2, suivant laquelle le sommet se situe adjacent au bord latéral dudit noyau absorbant (6).

4. Serviette sanitaire selon les revendications 1, 2 ou 3, suivant laquelle la largeur de ladite portion supérieure est essentiellement la même que celle de la portion inférieure.
